# EUROPEAN PATENT APPLICATION

(11) **EP 0 592 230 A1**
(43) Date of publication of application: **13.04.1994**
(21) Application number: 93308006.1
(22) Date of filing: 07.10.1993
(51) Int. Cl.: C07K 15/00, C12P 21/08, C07K 7/06, A61K 39/395

(54) **Human monoclonal anti-peptide anti-body and DNA encoding thereof**

(30) Priority: 07.10.1992 JP 293800/92
(71) Applicant: SNOW BRAND MILK PRODUCTS CO., LTD., Sapporo-shi, Hokkaido 065 (JP)
(72) Inventor: Washida, Naohiro, Shimotsuga-gun, Tochiga (JP); Yoshida, Toshiko, Utsunomiya-shi, Tochigi (JP); Morinaga, Tomonori, Ishibashimachi, Shimotsuga-gun, Tochigi (JP); Mizuno, Atsuko, Kiryu-shi, Gunma (JP); Goto,Masaaki, Shimotsuga-gun, Tochigi (JP); Kobayashi, Fumie, Kawachi-gun, Tochigi (JP)
(74) Representative: Boeters, Hans Dietrich, Dr.

(57) **Abstract**

Human monoclonal antibody directed against the peptide listed below was developed. The peptide exists in the CH4 region of human IgE and is related to signal transduction of chemical mediator release from sensitized mast cells and basophils.
H-Lys-Thr-Lys-Gly-Ser-Gly-Phe-Phe-Val-Phe-NH₂

The monoclonal antibody inhibits the histamine release from mast cells by stimulation with allergen. As the antibody thereof recognizes a specific amino acid sequence relates to allergic reactions, this antibody is useful as medicines and reagents.

## Description

### Background of the Invention

### Field of the Invention

This invention relates to a novel human type monoclonal antibody directed against a peptide having specific amino acid sequence which immunoglobulin E and is related to allergic reaction. This invention further relates to a DNA encoding the amino acid sequence of the antibody.

### Description of the Prior Art

Immunological reaction is a mechanism which defend the invasion of exogenous foreign materials such as infection. This reaction, however, may be sometimes harmful to the living body and is generally called allergy. The allergy is classified into Types I to IV on allergy mechanism. The Type I exhibits a typical allergic reaction as an immediate type hypersensitivity and allergy has become synonymous with Type I hypersensitivity. The occurrence of Type I allergy is mediated by immunoglobulin E (IgE). The onset mechanism includes the binding of IgE to Fcε receptor on the surfaces of mast cells in the tissues and basophils in the blood, followed by binding of allergen to IgE antibody to form cross linked structure between IgE antibodies. The cross-linking induces mast cells and basophils to release various chemical mediators, thus triggers a variety of allergic reactions such as asthma and edema.

With the elucidation of mechanism, therapeutic agents which react with IgE have been investigated for the prevention and treatment of allergy. Particularly, antibodies to IgE have been tried for the prophylaxis and therapy of allergy. Stanworth et al. found an amino acid sequence in CH4 region of IgE antibody, which is expected to stimulate histamine release from mast cells on the basis of analysis of signal transfer mechanism for the release of chemical mediators from mast cells and basophils (Stanworth, D.R., et. al., Biochem. J., 180, 665-668 (1979)). He also observed that a peptide having specific amino acid sequence shown below stimulated histamine release from mast cells in vitro. The amino acid sequence is identical to that of Sequence ID No. 1.

H-Lys-Thr-Lys-Gly-Ser-Gly-Phe-Phe-Val-Phe-NH2

Furthermore, they demonstrated the inhibition of histamine release from antigen stimulated rat mast cells with rabbit antiserum against the peptide both in vitro and in vivo test, and reported a probable new immunotherapy of allergic diseases using the peptide as a vaccine (Stanworth, D.R., et al., Lancet, 339, 1279-81 (1990)).

Human monoclonal antibody is most preferable in the application of antibody to the peptide for the treatment of allergic diseases. However, establishment of hybridoma cells producing human antibody was technically difficult and was not as popular as those of mice, and only few reported the success. For example, establishment of human hybridoma was first reported successively in 1980 by two groups of investigators (Olsson, L. and Kaplan, H.S., Proc. Natl. Acad. Sci., USA, 77, 5429 (1980) and Croce, C.M. et al., Nature, 288, 488 (1980)). Nonetheless, the yield of hybridomas which produce the aimed specific antibody was low and no technique had been developed in vitro production of the aimed antibody. Therefore many problems remained being unsolved in comparison to those of mice.

These problems are now under investigation and resolution, and some cell strains with high fusion efficiency, growth rate and stability have been obtained.

For example,
LICR-LON-HMy2 ---- Edwards, P.A.W., et al.,
Eur. J. Immunol., 12, 641 (1982),
WI-L2/729 HF₂ ---- Abrams, P.G., et al.,
J. Immunol., 131, 1201 (1983),
8226 AR/NIP4-1 ---- Pickering, J.W. and Gelder, F.B.,
J. Immunol., 129, 406 (1982), and
K6H6/B5 ---- Carrol, W.L., et al.,
J. Immunol. Methods, 89, 61 (1986)

Human lymphocytes can be easily obtained from peripheral blood as lymphocyte sources and also from spleen, tonsils and lymph nodes during operation.
Peripheral blood lymphocytes ---- Croce, C.M., et al.,
Nature, 288, 488 (1980),
Spleen ---- Olsson, L. and Kaplan, H.S.,
Proc. Natl. Acad. Sci., USA, 77, 5429 (1980), and
Tonsils ---- Edwards, P.A.W., et al.,
Eur. J. Immunol., 12, 641 (1982)

In vitro stimulation of in vivo sensitized lymphocytes is carried out by polyclonal activation of B cells with PWM or EBV, (PWM ---- Warenius, H.M., et al., Eur. J. Cancer Clin. Oncol., 19, 347 (1983), EBV ---- Kozbor, D. and Roder, J.C., Eur. J. Immunol., 14, 23 (1984)). It has been considered difficult to induce the aimed antibody only by in vitro stimulation of unsensitized B cells with antigen. However, Strike et al. reported the establishment of hybridoma cells producing antibodies to sheep erythrocytes by in vitro sensitization (Strike, L.E., et al., J. Immunol., 132, 1798 (1984)). No human monoclonal antibody against IgE provided by the present invention has been reported.

### Summary of the Invention

The antipeptide antibody of human origin is considered optimal mentioned above for the treatment of human allergic diseases, however, no such antibody has been found in literatures.

Therefore, one object of the present invention is to provide a novel human monoclonal antibody recognizing the peptide found by Stanworth et al. which exists in human IgE and participates in the trigger anaphylactic mediator release. Another object of the present invention is to provide a DNA encoding the amino acid sequence of the antibody.

The antibody of the present invention can be obtained by cell fusion of human lymphocytes in vitro sensitized by the peptide mentioned above with human myeloma cell lines to give hybridoma cells, followed by screening the specific hybridoma cells producing the aimed antibody. The resultant antibody producing hybridoma cells are used to obtain cDNA encoding the amino acid sequence of the antibody. The cDNA and the N-terminal amino acid sequence of the antibody are analyzed to determine the total amino acid sequence of human antibody. The antibody to the peptide has a specific amino acid sequence in the variable region and can be clearly distinguished from the other antibodies.

### Brief Description of the Drawings

Fig. 1 illustrates the HPLC pattern of synthetic peptide used as an antigen in the present invention.

Fig. 2 illustrates the binding of the antibody in culture supernatant of hybridoma and the peptide of the present invention.

Fig. 3 illustrates the base sequence of cDNA encoding the amino acid sequence of L-chain of the antibody and the amino acid sequence of the present invention.

Fig. 4 illustrates the base sequence of cDNA encoding the amino acid sequence of H-chain of the antibody and amino acid sequence of the present invention. (continue to Fig. 5)

Fig. 5 continues from Fig. 4 and illustrates the base sequence of cDNA encoding the amino acid sequence of H-chain of the antibody and amino acid sequence of the present invention.

Fig. 6 illustrates the results of the inhibition test by the anti-peptide antibody of histamine release from rat mast cells stimulated with the peptide.

### Detailed Description of Preferred Embodiments

The antibody of the present invention can be prepared by the following steps.
(a) Preparation of antigen
   The peptide is composed of 10 amino acid residues (Formula 1).
   The peptide is used for triggering chemical mediator release from mast cells and existing in the CH4 region of human IgE antibody is synthesized by Fmoc method using automatic peptide synthesizer 431A (Applied Biosystems Inc.) and purified by reverse phase HPLC. H-Lys-Thr-Lys-Gly-Ser-Gly-Phe-Phe-Val-Phe-NH2 (Formula 1)
   The purified peptide is conjugated to ovalbumin (OVA) using 1-ethyl-3-(dimethylaminopropyl)carbodiimide (EDC) and used as an antigen for in vitro sensitization.
(b) Preparation of hybridoma
   Human peripheral blood, spleen, tonsils and lymph nodes can be used as lymphocyte sources and these cells are immunized in vitro with the antigen and used for cell fusion with human myeloma cells. Suitable myeloma cell lines for fusion include LICR-LON-HMy2, WI-L2/729 HF₂, 8226 AR/NIP4-1, and K6H6/B5. The cell fusion is performed by a conventional method such as polyethylene glycol (PEG), Sendai virus and electric pulse methods.
(c) Screening of hybridoma cells
   The fused cells are chosen by cultivation in a selection medium. For example, the selection medium consists of culture medium supplemented with azaserine when K6H6/B5 is used as myeloma. The cell culture supernatants are screened for the desired monoclonal antibodies with ELISA, RIA, plaque assay and so forth.
(d) Culture of hybridoma
   The hybridomas can be expanded by inoculation into nude mouse or SCID mouse and the desired antibody can be purified from the ascite or serum. The antibody can also be prepared from culture supernatants of hybridoma cells by cultivating in RPMI-1640 medium containing 10% fetal calf serum or absence of the serum.
(e) Preparation of antibody
   The isolation and purification of the antibody from culture supernatants or ascites is carried out by conventional methods. For example, ammonium sulfate fractionation, gel filtration, ion exchange chromatography and affinity chromatography can be used singly or in combination if necessary.
(f) Characteristic features of the antibody
   The monoclonal antibody obtained by the method of the present invention is specified by the following characteristic features.

1. Binding to a synthetic peptide H-Lys-Thr-Lys-Gly-SerGly-Phe-Phe-Val-Phe-NH₂.
2. Inhibition of histamine release from mast cells stimulated with allergen.
3. Molecular weight of approximately 150,000 under non-reduced condition and classified to IgG3(κ) subclass of human IgG.

The recombinant antibody can be produced using DNA isolated from the antibody producing hybridoma of the present invention encoding the antibody by conventional methods. The present invention also provides single chain antibody and DNA which encode single chain antibodies. The antibody of the present invention is of human origin, thus can be safely and repeatedly administered to patients with allergic diseases. The antibody can be used for the treatment of diseases caused by allergic reaction to IgE such as hay fever, asthma, and so forth. The human type antibody allows intravenous administration and early treatment to immediate allergic reactions.

The present invention will be explained more in detail by the following examples.

### [Example 1]

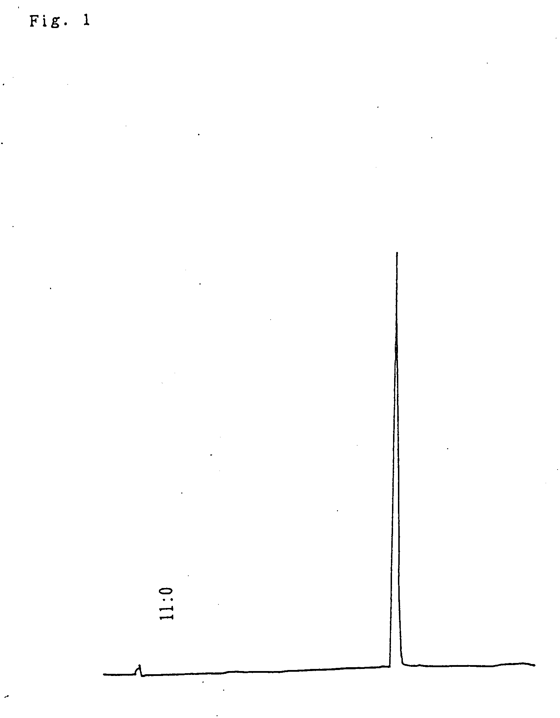

Preparation of human type peptide antibody productive hybridoma
(1) Preparation of antigen
   The peptide shown by Formula 1 and composed of 10 amino acid residues used as a releaser of chemical mediator from mast cells was synthesized by Fmoc method using automatic peptide synthesizer 431A (Applied Biosystems Inc.) from one mmole each of amino acid and 0.25 mmole of a resin. The synthesized peptide was cleaved from the resin by TFMSA method ('Introduction to Cleavage Techniques' published by Applied Biosystems) to give 130 mg of crude peptide. The crude peptide was purified with a reverse phase HPLC (Applied Cartridge Column RP-300, C8, ø 4.6 x 250 mm) to give 50 mg of the aimed peptide with purity of 99% or over. The chromatogram of the peptide is shown in Fig.1. The purified peptide was bound to ovalbumin using Imject Immunogen EDC Conjugation Kit (Pierce Co., Ltd.) and used as an antigen for in vitro immunization.
(2) Preparation of antigen sensitized lymphocytes
   Twenty milliliter of heparinized peripheral blood was drawn from a healthy volunteer and lymphocytes were isolated using Lymphosepar (Immune-Biological Laboratories). The isolated lymphocytes were suspended in RPMI-1640 medium, treated with leucine-O-methyl ester and sensitized in vitro with an antigen (1-10 µg of peptide-OVA conjugate) at 37°C for 20 min., then incubated at 37°C in a CO₂ incubator for four days in the presence of muramyl dipeptide, human IL4, IL6 and fetal calf serum (final concentration of 20%). Human myeloma cells K6H6/B5 were cultured by a conventional method using RPMI-1640 medium containing 10% fetal calf serum for the cell fusion with the above cells.
(3) Cell fusion
   Human lymphocytes and myeloma cells prepared above were mixed at a ratio of 2:1 in number of cells, centrifuged and the supernatants were removed. Then, one ml of 42% PEG4000-17% DMSO in RPMI-1640 medium pre-warmed at 37°C was added dropwisely to the cell pellets. To the mixed solution, 10 ml of RPMI-1640 medium without fetal calf serum (FCS) was added gradually with stirring, the mixture was centrifuged and the supernatants were removed and the cells were diluted to make 2-5 x 10⁶ cells/ml of lymphocytes with RPMI-1640 medium supplemented with 10% FCS. The cell suspension was distributed 0.1 ml/well each in a 96 well plate.
(4) Screening of hybridoma
   The cells were cultured for 10-14 days adding HT medium containing azaserine on days four, six and nine. The above mentioned HT medium containing azaserine was prepared by addition to make 0.1 mM of hypoxanthine, one µg/ml of azaserine, 1.6 µM of thymidine, 5 x 10⁻⁶ M of 2-mercaptoethanol, one ng/ml of human IL6, 100 U/ml of penicillin and 0.1 mg/ml of streptomycin to RPMI-1640 medium supplemented with 10% FCS. The screening of culture supernatants were performed by the following steps.
(5) Preparation of plates for screening
   In a 96 well plate (Nunc Co., Ltd.), 0.2 ml each of 2% bovine serum albumin was added and allowed to stand overnight at 4°C. The wells were washed and 0.1 ml each of PBS (pH 7.4) containing 10 µg/ml of the peptide and 0.25% glutaraldehyde was poured into wells and caused to react for one hr. at room temperature. The wells were washed, 0.2 ml each of 25 mM Tris buffer (pH 7.4) was poured and allowed to stand overnight at 4°C to prepare plates for screening.
(6) Screening of hybridoma
   Supernatants in wells confirmed the growth of cells were collected, poured 0.1 ml each to the above wells of plate for screening and allowed to stand for two hrs. at room temperature. The wells were washed three times with PBS-0.05% Tween 20 (PBS-T), 0.1 ml/well each of peroxidase labeled goat anti-human IgG antibody (DAKO Co., Ltd.) was added and incubated for two hrs. at room temperature. The wells were washed three times with PBS-T and 0.2 ml/well each of a solution, prepared from 20 ml of 0.1 M sodium acetate-0.05 M sodium dihydrogen phosphate, 1.0 ml of 40 mM ABTS (2,2'-azino-di-(3-ethylbenzothiazolin-sulfonate) and 0.2 ml of 0.25 M of H₂O₂, was added and a reaction was carried out at room temperature. After the reaction, the absorbance at 405 nm was determined with ImmunoReader NJ-2000 (Nippon InterMed Co., Ltd.).

The hybridoma cells which produce antibodies specifically react with the peptide were distributed into a 96 well plate at a rate of one cell/well were cloned three times by limiting dilution method. The characteristic features of antibody produced hybridomas were analyzed according to the following examples and the antibody was named 13-8G. The hybridoma was deposited to National Institute of Bioscience and Human-Technology; Agency of Industrial Science and Technology as FERM BP-4414.

### [Example 2]

### Culture of hybridoma cells and purification of antibody

Hybridoma cells were cultured in RPMI-1640 medium containing 10% FCS under 5% CO₂ atmosphere at 37°C in an incubator. The culture supernatants were harvested and grown cells were washed three times with PBS solution. The cells were suspended in serum-free RPMI-1640 medium at a rate of 1 x 10⁶ cells/ml and cultured at 37°C for three days in the CO₂ incubator. The serum-free culture supernatants were obtained by centrifugation.

Antibodies were purified from the culture supernatants containing FCS with ammonium sulfate fractionation and anti-human IgG antibody immobilized Sepharose (Cappel Co., Ltd.). Antibodies were purified specifically from the culture supernatants containing no FCS with protein G immobilized Sepharose (Zymed Co., Ltd.).

### [Example 3]

### Determination of physicochemical properties of monoclonal antibody

The antibody produced by hybridoma-clone obtained by the Example 1 was analyzed.
(1) Determination of molecular weight
   The determination was performed using SDS-polyacrylamide gel electrophoresis (SDS-PAGE) in Laemmli buffer. The molecular weight of the antibody was approximately 150,000 dalton under non-reduced condition by comparison using a molecular marker (BioRad Co., Ltd.).
(2) Isotype analysis of the antibody
   The analysis was performed using human IgG subclass typing kit (Binding Site Co., Ltd.) and the antibody produced by hybridoma clone 13-8G was classified to IgG3(κ) subclass.
(3) N-terminal amino acid sequence
   The purified antibody was dissolved in 10 mM Tris-HCl buffer (pH = 8) containing one mM EDTA, 2.5% of SDS, 0.01% of bromphenol blue, 10% 2-mercaptoethanol and 10% glycerol at a concentration of two µg/µl. The reaction mixture was heated at 100°C for three minutes and centrifuged at 15,000 rpm for three minutes to recover the supernatant. The supernatant was subjected to 10% SDS-PAGE to divide H- and L-chains. The chains were electrically blotted onto polyvinylidenedifluoride membrane, stained with Coomassie brilliant blue. The stained membrane was decolorized with 25% methanol containing 7% acetic acid and dried in the air. The area corresponding to the respective chain was cut out and directly introduced in a vapor phase protein sequencer (Model 477A, Applied Biosystems Inc.) to cause automatic coupling cleavage conversion. The resultant PTH-amino acids were dissolved in 20% acetonitrile, subjected to a reverse phase high performance liquid chromatography (Model 120A, column C-18, ø 2.1 mm x 220 mm, Applied Biosystems Inc.) and the respective amino acid was identified according to the retention time. The N-terminal amino acid sequence of L-chain of the antibody produced by clone 13-8G is shown below. The N-terminal amino acids of H-chain were blocked and could not analyze by this method.

The N-terminal amino acid sequence of L-chain of the antibody produced by clone 13-8G.

1 2 3 4 5 6 7 8 9 10 11 12 13 14 15 16 17 18 19 20
E I V M T Q S P A T L S V S P G G R A A

### [Example 4]

### Biochemical properties of monoclonal antibody

The antibody produced by hybridoma clone obtained by the Example 1 was analyzed.

### (1) The binding of monoclonal antibody to the peptide

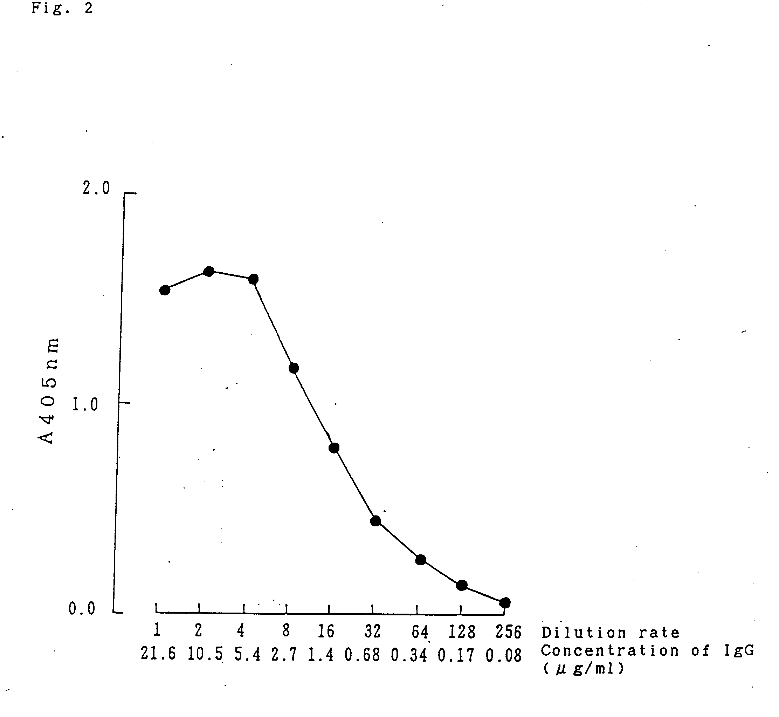

The binding of monoclonal antibody to the peptide was determined using ELISA method used in Example 1-(6). Concentration of human IgG in the culture supernatant was determined using EIA human IgG kit (MBL Co., Ltd.). In wells of a plate for analysis, 0.1 ml each of the culture supernatant diluted with 2%BSA-PBS solution was added and absorbances were determined by a similar method, shown in Fig. 2. The decrease of absorbance in proportion to the dilution of added hybridoma culture supernatant was observed confirming the dose dependent binding of the antibody with the peptide. Furthermore, the specific binding was found because of the use of 2% BSA for the dilution of culture supernatant.

### (2) cDNA cloning of antibody gene in clone 13-8G

Poly A tailed RNA was isolated from 1.2 x 10⁸ cells of hybridoma 13-8G strain using Fast-Track (InVitrogen Co., Ltd.). 1.7 µg of double strand of cDNA was synthesized using five µg of the isolated RNA. EcoRI adaptor was ligated at the both ends of the half amount of the cDNA and subjected to gel chromatography on Sepharose as a carrier. The resultant cDNA was inserted to λgt10 phage DNA and caused the package to give a λgt10 cDNA library.

Probes for screening were synthesized by PCR . A pair of PCR primers were synthesized according to the known base sequences of H- and L-chains of human IgG antibody and PCR amplification was carried out using the cDNA library as a template. The amplified DNA was purified using agarose electrophoresis, labeled with ³²P and used as a probe.

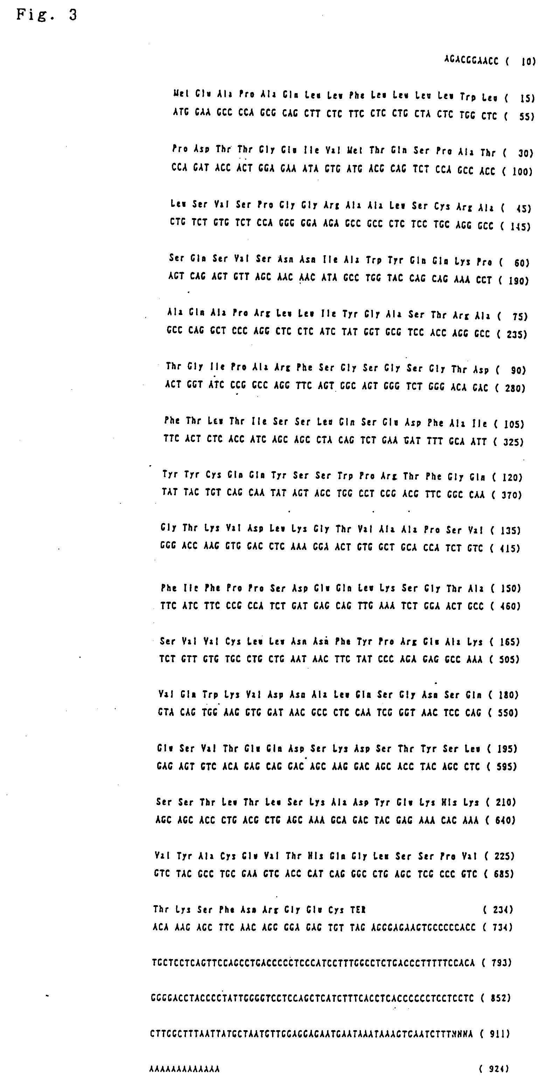

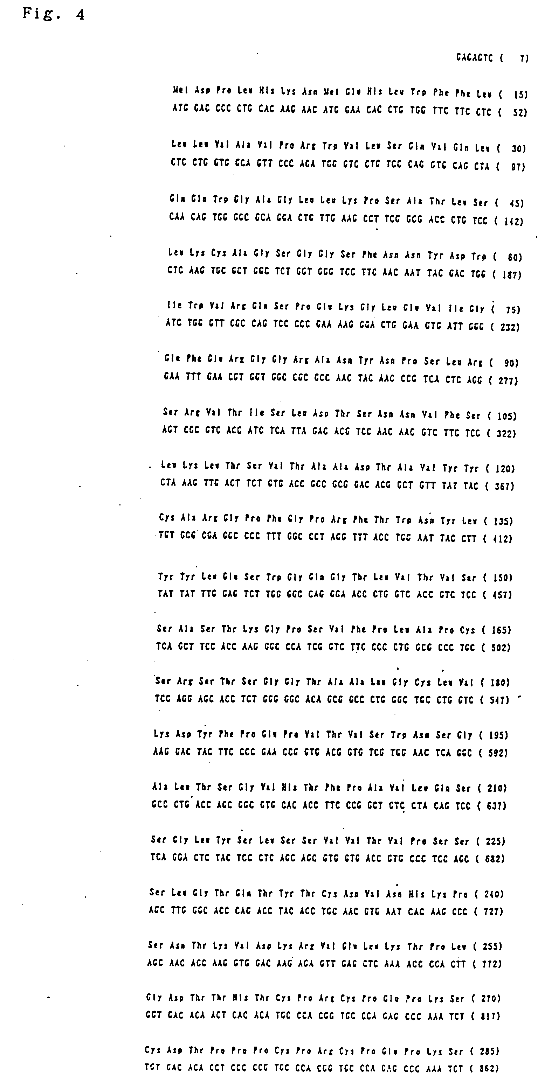

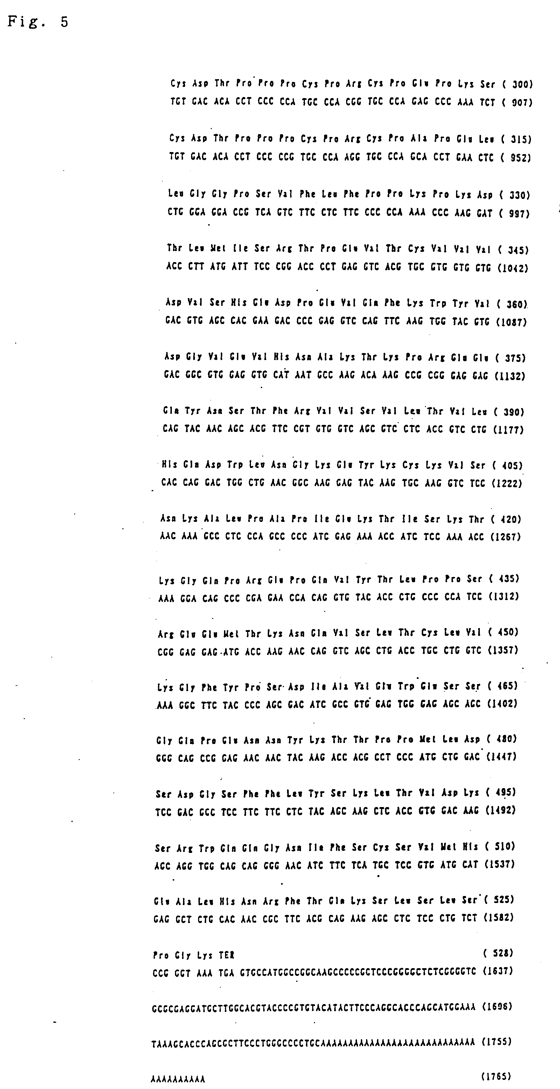

The probes of H- and L-chains were used for the screening of cDNA library and pure positive clones of K11 and H71 were selected. These clones were cut with restriction enzyme as EcoRI and BamHI to give fragments of 1.4-2.0 kb, the fragments were inserted into a plasmid vector, pBLUESCRIPT SK⁺, and subjected to subcloning. Colonies of Escherichia coli containing the antibody gene were screened by PCR to purify plasmid DNA. The plasmid was sequenced using DyeDeoxy™ Terminator Cycle Sequencing Kit (ABI). The DNA sequences are shown in Fig. 3, 4 and 5. Fig. 3 shows cDNA sequence of the L-chain and Figs. 4 and 5 (Figs. 4 and 5 show a serial cDNA sequence) show cDNA sequence in the H-chain. The symbol N represents unidentified three bases in non-coding region in the L-chain. The presumed amino acid sequence of H- and L-chains of the antibody are shown above the base sequences. The variable region in the H- and L-chains of the antibody were determined.

L-chain variable region:
Glu Ile Val Met Thr Gln Ser Pro Ala Thr Leu Ser Val Ser Pro
Gly Gly Arg Ala Ala Leu Ser Cys Arg Ala Ser Gln Ser Val Ser
Asn Asn Ile Ala Trp Tyr Gln Gln Lys Pro Ala Gln Ala Pro Arg
Leu Leu Ile Tyr Gly Ala Ser Thr Arg Ala Thr Gly Ile Pro Ala
Arg Phe Ser Gly Ser Gly Ser Gly Thr Asp Phe Thr Leu Thr Ile
Ser Ser Leu Gln Ser Glu Asp Phe Ala Ile Tyr Tyr Cys Gln Gln
Tyr Ser Ser Trp Pro Arg Thr Phe Gly Gln Gly Thr Lys Val Asp
Leu Lys Gly

H-chain variable region:
Gln Val Gln Leu Gln Gln Trp Gly Ala Gly Leu Leu Lys Pro Ser
Ala Thr Leu Ser Leu Lys Cys Ala Gly Ser Gly Gly Ser Phe Asn
Asn Tyr Asp Trp Ile Trp Val Arg Gln Ser Pro Glu Lys Gly Leu
Glu Val Ile Gly Glu Phe Glu Arg Gly Gly Arg Ala Asn Tyr Asn
Pro Ser Leu Arg Ser Arg Val Thr Ile Ser Leu Asp Thr Ser Asn
Asn Val Phe Ser Leu Lys Leu Thr Ser Val Thr Ala Ala Asp Thr
Ala Val Tyr Tyr Cys Ala Arg Gly Pro Phe Gly Pro Arg Phe Thr
Trp Asn Tyr Leu Tyr Tyr Leu Glu Ser Trp Gly Gln Gly Thr Leu
Val Thr Val Ser Ser

### (3) Histamine release inhibition by anti-peptide antibody from rat mast cells stimulated with the peptide

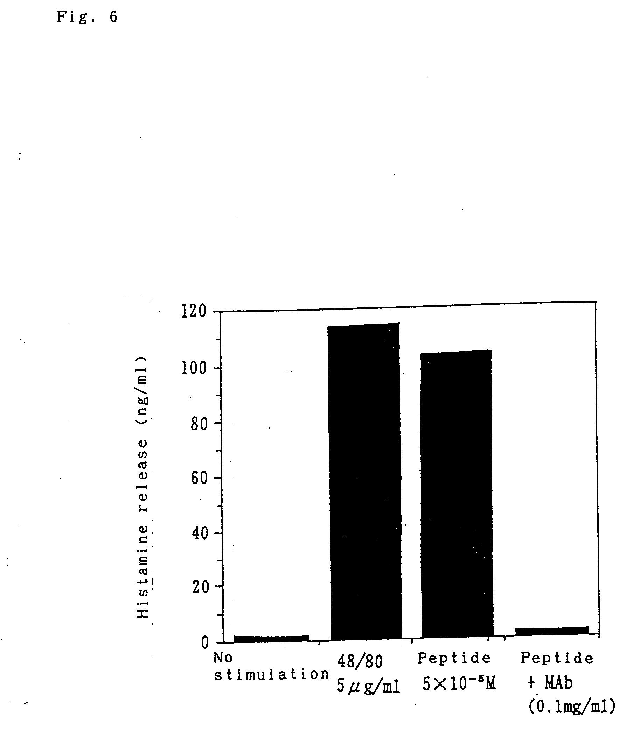

Intraperitoneal infiltrated cells of male Wistar rat, seven week old, were collected by a known method and used as mast cells. Histamine was released by a reaction of 1 x 10⁶ of mast cells and the peptide shown by Formula 1 at a concentration of 5 x 10⁻⁵ M and at 37°C for 30 min. The release was completely diminished by the addition of 0.1 mg/ml of the anti-peptide antibody exhibiting the inhibition of histamine release with the corresponding antibody. The results are shown in Fig. 6. The quantitative determination of histamine was performed with Histamine Release Test (Miles Co., Ltd.)

The present invention provides a human type monoclonal antibody and the DNA encoding the antibody which inhibits the signal transmission for the release of chemical mediator from mast cells and basophils stimulated with allergen. The antibody is a human type antibody with a definite antigen specificity. Its base sequence in the variable region, which express the antigen binding site is specified and this antibody can be used as medicines and reagents.

## Claims

**(1)** A human monoclonal antibody recognizes a peptide mentioned below which exists in human IgE and is related to signal of chemical mediator release from sensitized mast cells, and characterized by the inhibition of histamine release from mast cells stimulated with an allergen.

H-Lys-Thr-Lys-Gly-Ser-Gly-Phe-Phe-Val-Phe-NH2

**(2)** The human monoclonal antibody according to the Claim 1 having whole or partial sequence amino acid sequence mentioned below of variable region of H-chain and of L-chain.

L-chain variable region:
Glu Ile Val Met Thr Gln Ser Pro Ala Thr Leu Ser Val Ser Pro
Gly Gly Arg Ala Ala Leu Ser Cys Arg Ala Ser Gln Ser Val Ser
Asn Asn Ile Ala Trp Tyr Gln Gln Lys Pro Ala Gln Ala Pro Arg
Leu Leu Ile Tyr Gly Ala Ser Thr Arg Ala Thr Gly Ile Pro Ala
Arg Phe Ser Gly Ser Gly Ser Gly Thr Asp Phe Thr Leu Thr Ile
Ser Ser Leu Gln Ser Glu Asp Phe Ala Ile Tyr Tyr Cys Gln Gln
Tyr Ser Ser Trp Pro Arg Thr Phe Gly Gln Gly Thr Lys Val Asp
Leu Lys Gly

H-chain variable region:
Gln Val Gln Leu Gln Gln Trp Gly Ala Gly Leu Leu Lys Pro Ser
Ala Thr Leu Ser Leu Lys Cys Ala Gly Ser Gly Gly Ser Phe Asn
Asn Tyr Asp Trp Ile Trp Val Arg Gln Ser Pro Glu Lys Gly Leu
Glu Val Ile Gly Glu Phe Glu Arg Gly Gly Arg Ala Asn Tyr Asn

Pro Ser Leu Arg Ser Arg Val Thr Ile Ser Leu Asp Thr Ser Asn
Asn Val Phe Ser Leu Lys Leu Thr Ser Val Thr Ala Ala Asp Thr
Ala Val Tyr Tyr Cys Ala Arg Gly Pro Phe Gly Pro Arg Phe Thr
Trp Asn Tyr Leu Tyr Tyr Leu Glu Ser Trp Gly Gln Gly Thr Leu
Val Thr Val Ser Ser

**(3)** The human monoclonal antibody according to the Claim 1 having whole or partial below mentioned sequence of whole amino acid sequence of L-chain and whole amino acid sequence of H-chain of human monoclonal antibody.

L-chain amino acid sequence:
Glu Ile Val Met Thr Gln Ser Pro Ala Thr Leu Ser Val Ser Pro
Gly Gly Arg Ala Ala Leu Ser Cys Arg Ala Ser Gln Ser Val Ser
Asn Asn Ile Ala Trp Tyr Gln Gln Lys Pro Ala Gln Ala Pro Arg
Leu Leu Ile Tyr Gly Ala Ser Thr Arg Ala Thr Gly Ile Pro Ala
Arg Phe Ser Gly Ser Gly Ser Gly Thr Asp Phe Thr Leu Thr Ile
Ser Ser Leu Gln Ser Glu Asp Phe Ala Ile Tyr Tyr Cys Gln Gln
Tyr Ser Ser Trp Pro Arg Thr Phe Gly Gln Gly Thr Lys Val Asp
Leu Lys Gly Thr Val Ala Ala Pro Ser Val Phe Ile Phe Pro Pro
Ser Asp Glu Gln Leu Lys Ser Gly Thr Ala Ser Val Val Cys Leu
Leu Asn Asn Phe Tyr Pro Arg Glu Ala Lys Val Gln Trp Lys Val
Asp Asn Ala Leu Gln Ser Gly Asn Ser Gln Glu Ser Val Thr Glu
Gln Asp Ser Lys Asp Ser Thr Tyr Ser Leu Ser Ser Thr Leu Thr
Leu Ser Lys Ala Asp Tyr Glu Lys His Lys Val Tyr Ala Cys Glu
Val Thr His Gln Gly Leu Ser Ser Pro Val Thr Lys Ser Phe Asn
Arg Gly Glu Cys

H-chain amino acid sequence:
Gln Val Gln Leu Gln Gln Trp Gly Ala Gly Leu Leu Lys Pro Ser
Ala Thr Leu Ser Leu Lys Cys Ala Gly Ser Gly Gly Ser Phe Asn
Asn Tyr Asp Trp Ile Trp Val Arg Gln Ser Pro Glu Lys Gly Leu
Glu Val Ile Gly Glu Phe Glu Arg Gly Gly Arg Ala Asn Tyr Asn
Pro Ser Leu Arg Ser Arg Val Thr Ile Ser Leu Asp Thr Ser Asn
Asn Val Phe Ser Leu Lys Leu Thr Ser Val Thr Ala Ala Asp Thr
Ala Val Tyr Tyr Cys Ala Arg Gly Pro Phe Gly Pro Arg Phe Thr
Trp Asn Tyr Leu Tyr Tyr Leu Glu Ser Trp Gly Gln Gly Thr Leu
Val Thr Val Ser Ser Ala Ser Thr Lys Gly Pro Ser Val Phe Pro
Leu Ala Pro Cys Ser Arg Ser Thr Ser Gly Gly Thr Ala Ala Leu
Gly Cys Leu Val Lys Asp Tyr Phe Pro Glu Pro Val Thr Val Ser
Trp Asn Ser Gly Ala Leu Thr Ser Gly Val His Thr Phe Pro Ala
Val Leu Gln Ser Ser Gly Leu Tyr Ser Leu Ser Ser Val Val Thr
Val Pro Ser Ser Ser Leu Gly Thr Gln Thr Tyr Thr Cys Asn Val
Asn His Lys Pro Ser Asn Thr Lys Val Asp Lys Arg Val Glu Leu
Lys Thr Pro Leu Gly Asp Thr Thr His Thr Cys Pro Arg Cys Pro
Glu Pro Lys Ser Cys Asp Thr Pro Pro Pro Cys Pro Arg Cys Pro
Glu Pro Lys Ser Cys Asp Thr Pro Pro Pro Cys Pro Arg Cys Pro
Glu Pro Lys Ser Cys Asp Thr Pro Pro Pro Cys Pro Arg Cys Pro
Ala Pro Glu Leu Leu Gly Gly Pro Ser Val Phe Leu Phe Pro Pro
Lys Pro Lys Asp Thr Leu Met Ile Ser Arg Thr Pro Glu Val Thr
Cys Val Val Val Asp Val Ser His Glu Asp Pro Glu Val Gln Phe
Lys Trp Tyr Val Asp Gly Val Glu Val His Asn Ala Lys Thr Lys
Pro Arg Glu Glu Gln Tyr Asn Ser Thr Phe Arg Val Val Ser Val
Leu Thr Val Leu His Gln Asp Trp Leu Asn Gly Lys Glu Tyr Lys

Cys lys Val Ser Asn Lys Ala Leu Pro Ala Pro Ile Glu Lys Thr
Ile Ser Lys Thr Lys Gly Gln Pro Arg Glu Pro Gln Val Tyr Thr
Leu Pro Pro Ser Arg Glu Glu Met Thr Lys Asn Gln Val Ser Leu
Thr Cys Leu Val Lys Gly Phe Tyr Pro Ser Asp Ile Ala Val Glu
Trp Glu Ser Ser Gly Gln Pro Glu Asn Asn Tyr Lys Thr Thr Pro
Pro Met Leu Asp Ser Asp Gly Ser Phe Phe Leu Tyr Ser Lys Leu
Thr Val Asp Lys Ser Arg Trp Gln Gln Gly Asn Ile Phe Ser Cys
Ser Val Met His Glu Ala Leu His Asn Arg Phe Thr Gln Lys Ser
Leu Ser Leu Ser Pro Gly Lys

**(4)** DNA encoding the amino acid sequence according to the sequence list No. 2.

**(5)** DNA encoding the amino acid sequence according to the sequence list No. 3.


SEQUENCE LISTING

(1) GENERAL INFORMATION:

(i) APPLICANT:
(A) NAME: SNOW BRAND MILK PRODUCTS CO., LTD.
(B) STREET: 1-1, NAEBOCHO 6-CHOME
(C) CITY: HIGASHI-KU
(D) STATE: SAPPORO-SHI
(E) COUNTRY: JAPAN
(F) POSTAL CODE (ZIP): TOKYO

(ii) TITLE OF INVENTION: HUMAN MONOCLONAL ANTI-PEPTIDE ANTI-BODY AND
DNA ENCODING THEREOF

(iii) NUMBER OF SEQUENCES: 3

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(v) CURRENT APPLICATION DATA:
APPLICATION NUMBER: JP [?]93800/1992

(2) INFORMATION FOR SEQ ID NO: 1:

(i) SEQUENCE CHARACTERISTICS:
(A) LENGTH: 10 amino acids
(B) TYPE: amino acid
(D) TOPOLOGY: unknown

(ii) MOLECULAR TYPE: peptide

(xi) SEQUENCE DESCRIPTION: SEQ ID NO: 1:

Lys Thr Lys Gly Ser Gly Phe Phe Val Phe
1               5                   10

(2) INFORMATION FOR SEQ ID NO: 2:

(i) SEQUENCE CHARACTERISTICS:
(A) LENGTH: 234 amino acids
(B) TYPE: amino acid
(D) TOPOLOGY: unknown

(ii) MOLECULAR TYPE: protein

(xi) SEQUENCE DESCRIPTION: SEQ ID NO: 2:

Met Glu Ala Pro Ala Gln Leu Leu Phe Leu Leu Leu Leu Trp Leu Pro
-20                 -15                 -10                 -5
Asp Thr Thr Gly Glu Ile Val Met Thr Gln Ser Pro Ala Thr Leu Ser
            -1  +1              5                   10
Val Ser Pro Gly Gly Arg Ala Ala Leu Ser Cys Arg Ala Ser Gln Ser

                15                  20                  25
Val Ser Asn Asn Ile Ala Trp Tyr Gln Gln Lys Pro Ala Gln Ala Pro
        30                  35                  40
Arg Leu Leu Ile Tyr Gly Ala Ser Thr Arg Ala Thr Gly Ile Pro Ala
45                  50                  55                  60
Arg Phe Ser Gly Ser Gly Ser Gly Thr Asp Phe Thr Leu Thr Ile Ser
                65                  70                  75
Ser Leu Gln Ser Glu Asp Phe Ala Ile Tyr Tyr Cys Gln Gln Tyr Ser
            80                  85                  90
Ser Trp Pro Arg Thr Phe Gly Gln Gly Thr Lys Val Asp Leu Lys Gly
        95                  100                 105
Thr Val Ala Ala Pro Ser Val Phe Ile Phe Pro Pro Ser Asp Glu Gln
    110                 115                 120
Leu Lys Ser Gly Thr Ala Ser Val Val Cys Leu Leu Asn Asn Phe Tyr
125                 130                 135                 140
Pro Arg Glu Ala Lys Val Gln Trp Lys Val Asp Asn Ala Leu Gln Ser
                145                 150                 155
Gly Asn Ser Gln Glu Ser Val Thr Glu Gln Asp Ser Lys Asp Ser Thr
            160                 165                 170
Tyr Ser Leu Ser Ser Thr Leu Thr Leu Ser Lys Ala Asp Tyr Glu Lys
        175                 180                 185
His Lys Val Tyr Ala Cys Glu Val Thr His Gln Gly Leu Ser Ser Pro
    190                 195                 200
Val Thr Lys Ser Phe Asn Arg Gly Glu Cys
205                 210

(2) INFORMATION FOR SEQ ID NO: 3:

     (i) SEQUENCE CHARACTERISTICS:
          (A) LENGTH: 528 amino acids
          (B) TYPE: amino acid
          (D) TOPOLOGY: unknown

     (ii) MOLECULAR TYPE: protein


     (xi) SEQUENCE DESCRIPTION: SEQ ID NO: 3: